# EUROPEAN PATENT SPECIFICATION

(11) **EP 4 301 336 B1**
(45) Date of publication and mention of the grant of the patent: **13.08.2025**
(21) Application number: 22711095.4
(22) Date of filing: 03.03.2022
(51) Int. Cl.: A61K 9/08, A61K 47/10, A61K 47/36, A61K 36/09, A61K 36/185, A61K 36/258, A61K 36/28, A61K 36/324, A61K 36/41, A61K 36/481, A61K 36/53, A61K 36/66, A61K 36/88, A61K 36/9068

(54) **MEDICAL COMPOSITION FOR INHALATION**
MEDIZINISCHE ZUSAMMENSETZUNG ZUR INHALATION
COMPOSITION MÉDICALE DESTINÉE À ÊTRE INHALÉE

(30) Priority: 04.03.2021 IT 202100005051
(43) Date of publication of application: 10.01.2024
(73) Proprietor: Ape8 S.r.l., 21040 Origgio (VA) (IT); Natural Academy S.r.l., 22066 Mariano Comense (CO) (IT)
(72) Inventor: FERRI, Emanuele, 23876 Monticello Brianza, Lecco (IT); DELL'AGLI, Mario, 20133 Milano (IT)
(74) Representative: Long, Giorgio
(86) International application number: PCT/IB2022/051873
(87) International publication number: WO 2022/185242

(56) References cited:
- CN-A- 105 168 119
- CN-B- 108 785 242
- IT-A1- MI20 131 138

## Description

### Field of application of the invention

The present invention relates to a liquid medical composition for inhalation.

### Background art

The use of medical devices for inhalation is widespread, in particular for the treatment of respiratory system diseases.

There are essentially three types of known devices: spray inhalation devices, powder inhalation devices and aerosol devices. While the first two are mainly used for fast-acting active ingredients, often in emergency situations or in any case to alleviate symptoms quickly (antiasthmatic, antihistamine and the like), the aerosol devices are generally for relatively continuous use and with dispensing methods of several minutes.

The dispensing length and the fact that they are table devices make their use unpleasant to many subjects.

The use of electronic cigarettes, so-called "vaping", is now a widespread practice all over the world, at least partially replacing cigarette smoking.

Electronic cigarettes vaporize a liquid comprising, in addition to a basic composition adapted to provide a sufficiently dense body to the vapor released, also a certain amount of various types of flavorings and, in certain cases, of nicotine. The basic composition mostly consists of a mixture of propylene glycol and glycerol which cause, especially the former, a toxicity to the cells of the respiratory tract. Electronic cigarettes also belong to a category of tobacco products, and do not fall under that of medical devices. Patent document ITMI20131138 discloses liquids for inhalation comprising 1,3-propanediol and glycerin.

The use of an electronic cigarette device in dispensing medical substances in place of or in addition to the flavorings normally used, although convenient due to the portability thereof, would thus not be advisable, due to the toxicity of the basic composition.

Therefore, the problem faced by the present invention is to provide a portable dispensing system for inhalation (personal vaporizer) of medical substances which overcomes the drawbacks listed above.

### Summary of the invention

The aforesaid technical problem is substantially solved by a composition comprising the technical features set out in one or more of the appended claims, the definitions of which form an integral part of the present description for the purpose of sufficiency of description.

Therefore, the present invention relates to a medical composition for inhalation comprising the following components:
a) water 48-72% by weight
b) 1,3-propanediol 25-35% by weight
c) hyaluronic acid or a salt thereof 0.10-0.25% by weight
d) optionally, a preservative 0.25-0.4% by weight
e) optionally, sorbitol 0.3-1% by weight
f) one or more botanical active substances 1-30% by weight.

The invention further relates to a kit comprising one or more doses of the composition of the invention and an interchangeable cartomizer for personal vaporizer. Further features and advantages of the present invention will become more apparent from the indicative and thus non-limiting description of a preferred, but not exclusive embodiments of the invention.

### Detailed description of the invention

The present invention relates to a composition for medical inhalation comprising the following components:
a) water 48-72% by weight
b) 1,3-propanediol 25-35% by weight
c) hyaluronic acid or a salt thereof 0.10-0.25% by weight
d) optionally, a preservative 0.25-0.4% by weight
e) optionally, sorbitol 0.3-1% by weight
f) one or more botanical active substances 1-30% by weight.

The water is preferably purified water FU.

The 1,3-propanediol (component (b)) serves as a viscosifying agent and flavoring carrier.

Component c) is preferably sodium hyaluronate. The function of hyaluronic acid or the salts thereof is that of filming and viscosifying agent.

The preservative (component d)) is preferably sodium benzoate.

Sorbitol (component e)) has the function of enhancing flavorings and protecting the resistance (the so-called "coil"), the heating element of the personal vaporizer. The term "active substances", as used herein, refers to either isolated and/or purified active substances or mixtures thereof, or to an extract or mixture of extracts of a plant material of various origins (leaves, flowers, stems, roots, rhizomes, fruits, etc.) or of natural origin, which can contain mainly one or more active compounds and which can typically be contained in preparations of a mainly botanical nature of various kinds, such as alcoholic solutions, powders and the like. The active substances are selected according to the type of effect to be obtained with the composition of the invention.

In certain embodiments, the active substance consists of the same hyaluronic acid or salts thereof (component c)), for which an effect on diseases of the oropharyngeal cavity, such as aphthae and aphthous stomatitis, is recognized. In such a case, the active substances referred to in point (f) can be omitted.

The hyaluronic acid is preferably medium molecular weight hyaluronic acid between 1000 and 4000 KDa, or between 1500 and 2000 KDa.

In certain embodiments the one or more active substances include propolis. Propolis is a resinous substance which bees collect from the buds and bark of some trees, such as poplars, firs, elms and birches, which they then mix with saliva, wax and pollen. Propolis has immunostimulant, antibacterial and anti-inflammatory properties. A hydroalcoholic extract of propolis can be used for the purposes of the present invention.

When present, propolis is contained in the composition of the invention in an amount ranging from 1 to 7% by weight. The botanical preparations used as component f) are preferably chosen, depending on the therapeutic effect to be obtained, from:
- **preparations acting on anxiety and/or insomnia**
   *Valeriana officinalis* (Valerian)
   *Humulus lupulus* (Hop)
   *Lavandula angustifolia* (Lavender)
   *Passiflora incarnata* (Passionflower)
   *Melissa officinalis* (Melissa)
   *Matricaria chamomilla* (Chamomile)
   *Eschscholzia californica* (California poppy)
   *Centella asiatica* (Centella)
   *Ginkgo biloba* (Ginkgo)
   *Withania somnifera* (Indian ginseng)
   *Hypericum perforatum* (St. John's Wort)
- **preparations acting on depression**
   *Hypericum perforatum* (St. John's Wort)
   *Crocus sativus* (Saffron)
   *Salvia rosmarinus* (Rosemary)
   *Rhodiola rosea* (Golden root)
   *Cimicifuga racemosa* (Black cohosh)
   *Camellia sinensis* (Tea plant)
   *Coffea arabica* (Coffee)
   *Theobroma cacao* (Cocoa)
   *Paullinia cupana* (Guarana)
- **preparations acting on cognitive impairment**
   *Ginkgo biloba* (Ginkgo)
   *Bacopa monnieri* (Water hyssop)
   *Melissa officinalis* (Melissa)
- **preparations acting as stimulants**
   *Coffea arabica* (Coffee)
   *Camellia sinensis* (Tea plant)
   *Paullinia cupana* (Guarana)
   *Ilex paraguariensis* (Matè)
   *Cola spp.* (Cola)
   *Theobroma cacao* (Cocoa)
- **preparations acting on pain and inflammation**
   *Cannabis sativa* (Hemp)
   *Boswellia serrata* (Boswellia)
   *Harpagophytum procumbens* (Devil's Claw)
   *Tanacetum parthenium* (Feverfew)
   *Commiphora myrrha* (Myrrh)
   *Zingiber officinale* (Ginger)
   *Curcuma longa* (Turmeric)
- **preparations acting on colds and flu**
   *Pelargonium sidoides* (African geranium)
   *Spirea ulmaria* (meadowsweet)
   *Sambucus nigra* (Elder)
   *Andrographis paniculata* (Creat)
   *Allium sativum* (Garlic)
   *Thymus vulgaris* (Thyme)
   *Eucalyptus globulus* (Eucalyptus)
   *Pimpinella anisum* (Anise)
- **preparations acting on cough and bronchitis**
   *Althea officinalis* (Marsh-mallow)
   *Polygala spp.* (Polygala)
   *Grindelia robusta Nutt* (Gum Plant)
   *Plantago spp.* (Plantain)
   *Primula veris* (Cowslip)
   *Hedera helix* (Ivy)
- **preparations acting as adaptogens**
   *Panax ginseng* (Korean ginseng)
   *Eleutherococcus senticosus* (Devil's Bush or Siberian Ginseng)
   *Rhodiola rosea* (Golden root)
   *Schisandra chinensis* (Magnolia-vine)
   *Withania somnifera* (Indian ginseng)
   *Uncaria tomentosa* (Gambier)
   *Astragalus membranaceus* (Mongolian milkvetch)
   *Codonopsis pilosula* (Dangshen)
   *Lepidium meyenii* (Maca)
   *Ocimum tenuiflorum* (Holy basil)
   *Pfaffia paniculata* (Brazilian ginseng)
   *Phyllantus niruri* (Gale of the wind)
   *Piper longum* (Long pepper)
- **preparations acting as immunostimulants**
   *Echinacea spp.* (Echinacea)
   *Astragalus membranaceus* (Mongolian milkvetch)
   *Eleutherococcus senticosus* (Devil's Bush or Siberian Ginseng)
   *Panax ginseng* (Korean ginseng)
   *Panax quinquefolius* (American ginseng)
   *Allium sativum* (Garlic)
   *Uncaria tomentosa* (Gambier)
   *Ganoderma lucidum* (Reishi Mushroom)
   *Grifola frondosa* (Maitake Mushroom)
   *Coriolus versicolor* (Turkey Tail)
   *Lentinula edodes* (Shiitake Mushroom)
- **preparations acting on hypertension**
   *Allium sativum* (Garlic)
   *Olea europea* (Olive)
   *Crataegus monogyna* (Common
   Hawthorn)
   *Punica granatum* (Pomegranate)
   *Hibiscus sabdariffa* (Hibiscus)
   *Nigella sativa* (Black cumin)
   *Linum usitatissimum* (Flax)
- **preparations acting on chronic venous insufficiency**
   *Centella asiatica* (Centella)
   *Vaccinium myrtillus* (Bilberry)
   *Vitis vinifera* (Grape vine)
   *Pinus maritima* (Maritime pine)
   *Hamamelis virginiana* (Witch hazel)
- **preparations acting on hyperlipidemia**
   *Oryza sativa* fermented with *Monascus purpureus* (Thai glutinous rice)
   *Allium sativum* (Garlic)
   *Cynara scolymus* (Artichoke)
   *Trigonella foenum-graecum* (Fenugreek)
   *Citrus bergamia* (Bergamot)
   *Glycine max* (Soy)
   *Silybum marianum* (Milk thistle)
- **preparations acting on hyperglycemia**
   *Galega officinalis* (Galega)
   *Trigonella foenum-graecum* (Fenugreek)
   *Gymnema sylvestre* (Gymnema)
   *Ocimum tenuiflorum* (Holy basil)
   *Morus alba* (White mulberry)
   *Berberis spp.* (Barberry)
   *Cinnamomum verum* (Cinnamon)
   *Momordica charantia* (Bitter melon)
   *Allium cepa* (Onion)
   *Panax ginseng* (Korean ginseng)
- **preparations acting on excess weight and obesity**
   *Fucus vesiculosus* (Bladder wrack)
   *Garcinia cambogia* (Garcinia)
   *Hoodia gordonii* (Bushman's hat)
   *Caralluma fimbriata* (Caralluma)
   *Ilex paraguariensis* (Matè)
   *Paullinia cupana* (Guarana)
   *Amorphophallus konjac* (Konjac)
   *Gymnema sylvestre* (Gymnema)
   *Opuntia ficus-indica* (Prickly pear)
   *Mentha piperita* (Peppermint)
   *Capsicum annuum* (Chili pepper)
   *Zingiber officinale* (Ginger)
   *Cinnamomum verum* (Cinnamon)
- **preparations acting on oral cavity disorders**
   *Aloe vera gel* (Aloe)
   *Propolis* (Propolis)
   *Camellia sinensis* (Tea plant)
   *Curcuma longa* (Turmeric)
   *Theobroma cacao* (Cocoa)
   *Rubus occidentalis* (Black raspberry)
   Rubus *fruticosus* (Blackberry)
- **preparations acting on dyspepsia**
   *Gentian lutea* (Great yellow gentian)
   *Zingiber officinale* (Ginger)
   *Capsicum annuum* (Chili pepper)
   *Cannabis sativa* (Hemp)
   *Mentha piperita* (Peppermint)
   *Citrus limon* (Lemon)
   *Foeniculum vulgare* (Fennel)
   *Carum carvi* (Caraway)
   *Glycyrrhiza glabra* (Licorice)
- **preparations acting on constipation and diarrhea**
   *Fraxinus ornus* (Manna ash)
   *Tamarindus indica* (Tamarind)
   *Aloe vera* (Aloe)
   *Rheum palmatum* (Rhubarb)
   *Cassia angustifolia* (Senna)
   *Rhamnus purshiana* (Cascara)
   *Rhamnus frangula* (Alder Buckthorn)
   *Agrimonia eupatoria* (Common Agrimony)
   *Potentilla anserina* (Silverweed)
   *Potentilla erecta* (Tormentil)
   *Alchemilla vulgaris* (Lady's mantle)
   *Quercus spp.* (Oak)
   *Rubus ulmifolius* (Wild blackberry)
- **preparations acting on intestinal inflammatory pathologies**
   *Mentha piperita* (Peppermint)
   *Curcuma longa* (Turmeric)
   *Boswellia serrata* (Boswellia)
   *Andrographis paniculata* (Creat)
- **preparations acting as hepatoprotective**
   *Silybum marianum* (Milk thistle)
   *Phyllantus niruri* (Gale of the wind)
   *Glycyrrhiza glabra* (Licorice)
   *Cynara scolymus* (Artichoke)
- **preparations acting as choleretics and cholagogues**
   *Curcuma longa* (Turmeric)
   *Cynara scolymus* (Artichoke)
   *Peumus boldus* (Boldo)
   *Taraxacum officinale* (Dandelion)
- **preparations acting on water retention**
   *Betula pendula* (Silver Birch)
   *Equisetum arvense* (Horsetail)
   *Cynodon dactylon* (Bermuda grass)
   *Hieracium pilosella* (Mouse-ear hawkweed)
   *Juniperus communis* (Juniper)
- **preparations acting on urinary tract infections**
   *Vaccinium macrocarpon*
   (American cranberry)
   *Arctostaphylos uva-ursi*
   (Bearberry)
- **preparations acting on benign prostatic hyperplasia**
   *Serenoa repens* (Saw palmetto)
   *Prunus africana* (African cherry)
   *Urtica dioica*/*urens* (Nettle)
   *Cucurbita pepo* (Pumpkin)
   *Epilobium spp* (Willowherbs)
   *Solanum lycopersicum* (Tomato)
   *Pinus maritima* (Maritime pine)
   *Vaccinium macrocarpon* (American cranberry)
   *Allium sativum* (Garlic)
   *Lepidium meyenii* (Maca)
   *Telfairia occidentalis* (Fluted gourd)
   *Uvaria rufa* (Susung-kalabaw or Carabao teats)
- **preparations acting on erectile dysfunction**
   *Panax ginseng* (Korean ginseng)
   *Lepidium meyenii* (Maca)
   *Tribulus terrestris* (Goat's head)
   *Cinnamomum cassia* (Cinnamon)
- **preparations acting on menopausal disorders**
   *Cimicifuga racemosa* (Black cohosh)
   *Glycine max* (Soy)
   *Trifolium pratense* (Red clover)
   *Vitex agnus-castus* (Vitex)
   *Linum usitatissimum* (Flax)
   *Hypericum perforatum* (St. John's Wort)
   *Panax ginseng* (Korean ginseng)
   *Ginkgo biloba* (Ginkgo)
   *Crocus sativus* (Saffron)
   *Valeriana officinalis* (Valerian)
- **preparations acting on the cessation of cigarette smoking**
   *Cimicifuga racemosa* (Black cohosh)
   *Laburnum anagyroides* (Laburnum)

The botanical preparations used as component f) of the composition of the invention are more preferably selected from:
- Devil's Claw (*Harpagophytum procumbens* DC. and/or *Harpagophytum zeyheri* Decne);
- Mongolian milkvetch *(Astragalus membranaceus);*
- Chamomile *(Matricaria chamomilla* (L.) All.);
- *Echinacea angustifolia* DC.;
- *Echinacea purpurea (L.);*
- Devil's bush *(Eleutherococcus senticosus* (Rupr. et Maxim.));
- California poppy *(Escholzia californica* Cham.);
- Ginseng (*Panax ginseng* C.A. Meyer);
- Lavender *(Lavandula angustifolia* Miller);
- Iceland lichen *(Cetraria islandica* (L.) Acharius s.l.);
- Hops *(Humulus lupulus L.);*
- Melissa *(Melissa officinalis L.);*
- Myrrh *(Commiphora molmol* Engler);
- Passionflower *(Passiflora incarnata* L.);
- Golden root *(Rhodiola rosea L.);*
- Ginger *(Zingiber officinale* Roscoe).

Hydroalcoholic, aqueous or dry extracts can be used, such as Devil's Claw and Saffron.

Devil's Claw can be used in the treatment of joint pain and digestive disorders or in the event of lack of appetite.

Mongolian milkvetch can be used as an immunostimulant and for the tonic action thereof.

Chamomile and Melissa can be used in the treatment of excessive gastrointestinal motility.

*Echinacea angustifolia* can be used as an immunostimulant, typically in the treatment of colds.

*Echinacea purpurea* can be used as an immunostimulant in cooling diseases or in the treatment of acne.

Devil's bush, Golden root and Korean Ginseng can be used in the treatment of asthenia, fatigue and weakness. California poppy, Lavender, Melissa, Passionflower and Hops can be used in the treatment of mental stress and as sleep aids.

Iceland lichen can be used in the treatment of oropharyngeal irritation, in the treatment of dry cough and as an appetite aid.

Myrrh can be used to treat inflammation and mouth ulcers (stomatitis and gingivitis) and to treat small wounds and boils.

Ginger can be used in the treatment of kinetosis and excessive gastrointestinal motility.

When used, the botanical extracts of the invention are included in the composition in an amount between 0.5 and 10% by weight. More in particular, the following botanical preparations will be contained in the following weight percentages:

| | |
|---|---|
| - Devil's claw | 1-7% |
| - Mongolian milkvetch | 1-7% |
| - Chamomile | 1-9% |
| *- Echinacea angustifolia* DC. | 1-7% |
| *- Echinacea purpurea* | 1-7% |
| - Devil's bush | 1-7% |
| - California poppy | 2-9% |
| - Ginseng | 1-7% |
| - Lavender | 2-9% |
| - Lavender flower | 2-9% |
| - Iceland lichen | 1-7% |
| - Hops | 2-9% |
| - Melissa | 2-9% |
| - Myrrh | 1-7% |
| - Passionflower | 2-9% |
| - Golden root | 2-9% |
| - Ginger | 1-7% |
| - Saffron | 1-5%. |

In an embodiment, the composition of the invention is a mixture of the following components (weight percentages): - purified water 48-55%

| | |
|---|---|
| - 1,3-propanediol | 25-35% |
| - sodium hyaluronate | 0.1-0.2% |
| - sorbitol | 0.4-0.6% |
| - sodium benzoate | 0.25-0.4% |
| - Lavender | 5-7% |
| - Hops | 5-7% |
| - Golden root | 5-7% |
| - Saffron | 2-3%. |

The above composition can be used as an adjuvant in subjects with anxiety, mental stress and mood disorders. In a different embodiment, the composition of the invention is a mixture of the following components (weight percentages):

| | |
|---|---|
| - purified water | 48-55% |
| - 1,3-propanediol | 25-35% |
| - sodium hyaluronate | 0.1-0.2% |
| - sorbitol | 0.4-0.6% |
| - sodium benzoate | 0.25-0.4% |
| - Passionflower | 4-6% |
| - Melissa | 4-6% |
| - California poppy | 4-6% |
| - Hops | 4-6%. |

The aforesaid composition can be used as an adjuvant in subjects with sleep disorders (insomnia, even when linked to stress, anxiety, seniority).

In a different embodiment, the composition of the invention is a mixture of the following components (weight percentages):

| | |
|---|---|
| - purified water | 48-55% |
| - 1,3-propanediol | 25-35% |
| - sodium hyaluronate | 0.1-0.2% |
| - sorbitol | 0.4-0.6% |
| - sodium benzoate | 0.25-0.4% |
| - Propolis | 3-5% |
| - Ginseng | 3-5% |
| *- Echinacea purpurea* | 3-5% |
| - Devil's bush | 3-5% |
| - Mongolian milkvetch | 3-5%. |

The aforesaid composition can be used as an immunostimulant, for example in subjects predisposed or professionally exposed to seasonal infectious diseases, even relapsing, and as a tonic.

In a different embodiment, the composition of the invention is a mixture of the following components (weight percentages):

| | |
|---|---|
| - purified water | 48-55% |
| - 1,3-propanediol | 25-35% |
| - sodium hyaluronate | 0.1-0.2% |
| - sorbitol | 0.4-0.6% |
| - sodium benzoate | 0.25-0.4% |
| - Propolis | 3-5% |
| - Myrrh | 3-5% |
| - Ginger | 3-5% |
| - Iceland lichen | 3-5% |
| - Devil's claw | 3-5%. |

The aforesaid composition can be used as an anti-inflammatory and antimicrobial agent, for example in subjects predisposed or professionally exposed to seasonal infectious diseases, even relapsing, in subjects with oral disorders and in elderly subjects.

### EXPERIMENTAL SECTION

To study the potential skin irritation of the compositions of the invention, a composition sample was applied as such on reconstructed human epidermis (RHE) of which cell viability was then assessed by MTT assay.

The reconstructed human epidermis consists of normal human keratinocytes cultured on an inert polycarbonate filter at the air-liquid interface in a chemically well-defined growth medium.

The chemical basis of the test is the reduction of MTT, a yellow substance in solution, to form purple formazan crystals. Such a reduction process mainly occurs in the mitochondria and is highly dependent on the activity of the succinic mitochondrial enzyme dehydrogenase. As a result of these metabolic processes, purple crystals of formazan appear within a few hours and can be dissolved in isopropanol. The absorbance of the solubilized crystals can be measured at a wavelength of 540 nm and is proportional to the number of living cells in a very wide linear range.

The reduction in cell viability of the tissues treated with the test sample with respect to the negative control is used to predict the potential for skin irritation.

For this purpose, 3 RHE tissues were treated with the tested product and 3 were treated with 5% sodium dodecyl sulfate (SDS) (positive control). As a negative control, 3 untreated RHE tissues (maintained in D-PBS during the incubation period) were used. The tested sample is considered irritating for the skin if the cell viability of the tissues after treatment is ≤50%.

The test was carried out following the UNI EN ISO 10993-10:2013 guidelines.

### Pre-incubation

The RHE tissues were left in growth medium for at least 2 hours (37°C, 5% CO₂) prior to treatment.

### Treatment

The RHE tissues were treated for 15 minutes with 30 µl of substance according to the following scheme:
- Negative control DPBS
- Positive control SDS 5%
- Tested sample.

Each experiment was conducted in triplicate.

### Washes

The RHE tissues were repeatedly washed with DPBS, in order to eliminate any trace of the substances used for the treatments.

### Post-incubation

The RHE tissues were transferred to growth medium for 42 ± 2 hours (37°C, 5% CO₂).

### MTT assay

The RHE tissues were treated with MTT (1 mg/ml) for 3 hours ± 5 minutes (37°C, 5% CO₂) and then the formazan crystals were extracted in isopropanol for 2 hours ± 5 minutes at room temperature. The optical density reading was performed at 540 nm.

The following samples were tested:

### - Sample A

50% PURIFIED WATER F.U. 30% 1,3-PROPANEDIOL (PDO), 0.17% ORAL GRADE SODIUM HYALURONATE (mg, powder), E 420 0.5% SORBITOL FU-Ph.Eur. (mg, powder), EP-E 211 0.3% GRANULAR SODIUM BENZOATE (mg, powder), 5.83% HYDROALCOHOLIC LAVENDER F.E., 5.83% HYDROALCOHOLIC HOPS F.E., 5.83% HYDROALCOHOLIC GOLDEN ROOT F.E, 2.5% SAFFRON D.E. (mg, powder) .

### - Sample B

50% PURIFIED WATER F.U. 30% 1,3-PROPANEDIOL (PDO), 0.15% ORAL GRADE SODIUM HYALURONATE (mg, powder), E 420 0.5% SORBITOL FU-Ph.Eur. (mg, powder), EP-E 211 0.3% GRANULAR SODIUM BENZOATE (mg, powder), 4% HYDROALCOHOLIC PROPOLIS F.E., 4% HYDROALCOHOLIC MYRRH F.E., 4% HYDROALCOHOLIC GINGER F.E., 4% HYDROALCOHOLIC ICELAND LICHEN F.E., 4% DEVIL'S CLAW D.E.

The MTT test gave the following vitality results of the treated tissues:

| | |
|---|---|
| - Sample A | 100.8% |
| - Sample B | 88.9%. |

The tested samples were thus non-irritating,

In particular, the data show that the basic composition, i.e., the one without botanical preparations, is totally free of cytotoxicity (composition A containing the basic composition with botanical extracts in addition) and that a slight but negligible irritant effect - much lower than the prefixed threshold - can be introduced as a function of the nature of the extracts used.

Therefore, the composition of the invention allows providing a system for administering active substances by inhalation by means of a portable device, preferably an inhalation device, eliminating the cytotoxicity problems related to the use of electronic cigarettes.

This result is achieved both by the use of a cytotoxicity-free basic composition (by virtue of the elimination of propylene glycol and glycerol), and with the addition of specific active substances, in particular botanical or propolis preparations or extracts, having low or no cytotoxicity.

The present invention departs from the state of the art by having developed a safe solution from a toxicological point of view, effective from a therapeutic point of view and at the same time functional from the point of view of compatibility with the electronic devices on the market. Toxicological safety (a fundamental requirement for medical applications) has been achieved by virtue of the inclusion of a high presence of water and the absence of substances capable of generating toxic compounds by pyrolysis. In fact, the water-based mixture is intended to be used by means of a personal vaporizer which, by heating the mixture, produces an inhalable aerosol. Water is used in high amounts (48 to 72% by weight) for the main purpose of increasing the biocompatibility of the emissions which will contain very low amounts of toxic byproducts and to keep the operating temperatures of the atomizers low.

However, the high amount of water makes the mixture incompatible with normal vaporizers on the market which normally tolerate percentages of water not exceeding 10%. This problem was solved by calibrating a precise quantitative ratio between water and hyaluronic acid. In fact, the latter, being a high molecular weight polymer able to bind to water molecules with great affinity, tends to create a gel in favorable circumstances. Suitably modulated, this feature allows simultaneously preventing water from crossing the resistances and inhibiting the correct operation of the atomizers, keeping the operating temperature low, and preventing hyaluronic acid from undergoing pyrolysis phenomena.

In summary, this aerosol has therapeutic properties by virtue of several concomitant features:
- The high biocompatibility due to the high presence of water;
- The high compatibility with atomizers on the market by virtue of the gel effect produced by hyaluronic acid with water;
- The high safety profile due to the low operating temperature of the atomizers;
- The high bioavailability of substances due to the abundant presence of water and the high uptake by the lungs.

The invention further relates to a kit comprising one or more doses of the composition of the invention and an interchangeable cartomizerfor personal vaporizer (containing the so-called "coil" or resistance) so as to provide an efficient composition dispensing system uncontaminated by foreign substances.

## Claims

1. A medical composition for inhalation comprising the following components:
| | |
|---|---|
| a) water | 48-72% by weight |
| b) 1,3-propanediol | 25-35% by weight |
| c) hyaluronic acid or a salt thereof | |
| | 0.10-0.25% by weight |
| d) optionally, a preservative | 0.25-0.4% by weight |
| e) optionally, sorbitol | 0.3-1% by weight |
| f) one or more botanical active substances | 1-30% by weight. |

2. The composition according to claim 1, wherein the hyaluronic acid or a salt thereof has a molecular weight between 1000 and 4000 KDa.

3. The composition according to any one of claims 1 to 2, wherein the one or more active substances are botanical extracts or preparations of one or more of the following plants:
- **preparations acting on anxiety and/or insomnia**
*Valeriana officinalis* (Valerian)
*Humulus lupulus* (Hop)
*Lavandula angustifolia* (Lavender)
*Passiflora incarnata* (Passionflower)
*Melissa officinalis* (Melissa)
*Matricaria chamomilla* (Chamomile)
*Eschscholzia californica* (California poppy)
*Centella asiatica* (Centella)
*Ginkgo biloba* (Ginkgo)
*Withania somnifera* (Indian ginseng)
*Hypericum perforatum* (St. John's Wort)
- **preparations acting on depression**
*Hypericum perforatum* (St. John's Wort)
*Crocus sativus* (Saffron)
*Salvia rosmarinus* (Rosemary)
*Rhodiola rosea* (Golden root)
*Cimicifuga racemosa* (Black cohosh)
*Camellia sinensis* (Tea plant)
*Coffea arabica* (Coffee)
*Theobroma cacao* (Cocoa)
*Paullinia cupana* (Guarana)
- **preparations acting on cognitive impairment**
*Ginkgo biloba* (Ginkgo)
*Bacopa monnieri* (Water hyssop)
*Melissa officinalis* (Melissa)
- **preparations acting as stimulants**
*Coffea arabica* (Coffee)
*Camellia sinensis* (Tea plant)
*Paullinia cupana* (Guarana)
*Ilex paraguariensis* (Matè)
*Cola acuminata* (Cola)
*Theobroma cacao* (Cocoa)
- **preparations acting on pain and inflammation**
*Cannabis sativa* (Hemp)
*Boswellia serrata* (Boswellia)
*Harpagophytum procumbens* (Devil's Claw)
*Tanacetum parthenium* (Feverfew)
*Commiphora myrrha* (Myrrh)
*Zingiber officinale* (Ginger)
*Curcuma longa* (Turmeric)
- **preparations acting on colds and flu**
*Pelargonium sidoides* (African geranium) or
*Spirea ulmaria* (Meadowsweet)
*Sambucus nigra* (Elder)
*Andrographis paniculata* (Creat)
*Allium sativum* (Garlic)
*Thymus vulgaris* (Thyme)
*Eucalyptus globulus* (Eucalyptus)
*Pimpinella anisum* (Anise)
- **preparations acting on cough and bronchitis**
*Althea officinalis*
(Marsh-mallow)
*Polyigala spp.*
(Polygala)
*Grindelia robusta Nutt*
(Gum Plant)
*Plantago spp.* (Plantain)
*Primula veris* (Cowslip)
*Hedera helix* (Ivy)
- **preparations acting as adaptogens**
*Panax Ginseng* (Korean ginseng)
*Eleutherococcus senticosus* (Devil's Bush or Siberian Ginseng) or
*Rhodiola rosea* (Golden root)
*Schisandra chinensis* (Magnolia-vine)
*Withania somnifera* (Indian ginseng)
*Uncaria tomentosa* (Gambier)
*Astragalus membranaceus* (Mongolian milkvetch)
*Codonopsis pilosula* (Dangshen)
*Lepidium meyenii* (Maca)
*Ocimum tenuiflorum* (Holy basil)
*Pfaffia paniculata* (Brazilian ginseng)
*Phyllantus niruri* (Gale of the wind)
*Piper longum* (Long pepper)
- **preparations acting as immunostimulants**
*Echinacea spp.* (Echinacea)
*Astragalus membranaceus* (Mongolian
milkvetch)
*Eleutherococcus senticosus* (Devil's
Bush or Siberian Ginseng)
*Panax ginseng* (Korean ginseng)
*Panax quinquefolius* (American ginseng)
*Allium sativum* (Garlic)
*Uncaria tomentosa* (Gambier)
*Ganoderma lucidum* (Reishi Mushroom)
*Grifola frondosa* (Maitake Mushroom)
*Coriolus versicolor* (Turkey Tail)
*Lentinula edodses* (Shiitake Mushroom)
- **preparations acting on hypertension**
*Allium sativum* (Garlic)
*Olea europea* (Olive)
*Crataegus monogyna* (Common Hawthorn)
*Punica granatum* (Pomegranate)
*Hibiscus sabdariffa* (Hibiscus)
*Nigella sativa* (Black cumin)
*Linum usitatissimum* (Flax)
- **preparations acting on chronic venous insufficiency**
*Centella asiatica* (Centella)
*Vaccinium myrtillus* (Bilberry)
*Vitis vinifera* (Grape vine)
*Pinus maritima* (Maritime pine)
*Hamamelis virginiana* (Witch hazel)
- **preparations acting on hyperlipidemia**
*Oryza sativa* fermented with *Monascus purpureus* (Thai glutinous rice)
*Allium sativum* (Garlic)
*Cynara scolymus* (Artichoke)
*Commiphora mukul* (Gugal)
*Trigonella foenum-graecum* (Fenugreek)
*Citrus bergamia* (Bergamot)
*Glycine max* (Soy)
*Silybum marianum* (Milk thistle)
- **preparations acting on hyperglycemia**
*Galega officinalis* (Galega)
*Trigonella foenum-graecum* (Fenugreek)
*Gymnema sylvestre* (Gymnema)
*Ocimum tenuiflorum* (Holy basil)
*Morus alba* (White mulberry)
*Berberis spp.* (Barberry)
*Cinnamomum verum* (Cinnamon)
*Momordica charantia* (Bitter melon) or
*Allium cepa* (Onion)
*Panax ginseng* (Korean ginseng)
- **preparations acting on excess weight and obesity**
*Fucus vesiculosus* (Bladder wrack)
*Garcinia cambogia* (Garcinia)
*Hoodia gordonii* (Bushman's hat)
*Caralluma fimbriata* (Caralluma)
*Ilex paraguariensis* (Matè)
*Paullinia cupana* (Guarana)
*Amorphophallus konjac* (Konjac)
*Gymnema sylvestre* (Gymnema)
*Opuntia ficus-indica* (Prickly pear)
*Mentha piperita* (Peppermint)
*Capsicum annuum* (Chili pepper)
*Zingiber officinale* (Ginger)
*Cinnamomum verum* (Cinnamon)
- **preparations acting on oral cavity disorders**
*Aloe vera gel* (Aloe)
*Camellia sinensis* (Tea plant)
*Curcuma longa* (Turmeric)
*Theobroma cacao* (Cocoa)
*Rubus occidentalis* (Black raspberry)
- **preparations acting on dyspepsia**
*Gentian lutea* (Great yellow gentian)
*Zingiber officinale* (Ginger)
*Capsicum annuum* (Chili pepper)
*Cannabis sativa (Hemp)*
*Mentha piperita* (Peppermint)
*Citrus limon* (Lemon)
*Foeniculum vulgare* (Fennel)
*Carum carvi* (Caraway)
*Glycyrrhiza glabra* (Licorice)
- **preparations acting on constipation and diarrhea**
Gum karaya
Guar Gum
*Fraxinus ornus* (Manna ash)
*Tamarindus indicates* (Tamarind) or
*Aloe vera* (Aloe)
*Rheum palmatum* (Rhubarb)
*Cassia angustifolia* (Senna)
*Rhamnus purshiana* (Cascara sagrada)Cascara
*Rhamnus frangula* (Alder Buckthorn)
*Agrimony eupatoria* (Common Agrimony) or
*Potentilla anserina* (Silverweed)
*Potentilla erecta* (Tormentil)
*Alchemilla vulgaris* (Lady's mantle)
*Quercus spp.* (Oak)
*Rubus ulmifolius* (Wild blackberry)
- **preparations acting on intestinal inflammatory pathologies, irritable colon**
*Mentha piperita* (Peppermint)
- **preparations acting on chronic intestinal inflammatory diseases**
*Curcuma longa* (Turmeric)
*Boswellia serrata* (Boswellia)
*Andrographis paniculata* (Creat)
- **preparations acting as hepatoprotective**
*Silybum marianum* (Milk thistle)
*Phyllantus niruri* (Gale of the wind)
*Glycyrrhiza glabra* (Licorice)
*Cynara scolymus* (Artichoke)
- **preparations acting as choleretics and cholagogues**
*Curcuma longa* (Turmeric)
*Cynara scolymus* (Artichoke)
*Peumus boldus* (Boldo)
*Taraxacum officinale*
(Dandelion)
- **preparations acting on water retention**
*Betula pendula* (Silver
Birch)
*Equisetum arvense*
(Horsetail)
*Cynodon dactylon*
(Bermuda grass)
*Hieracium pilosella*
(Mouse-ear hawkweed)
*Juniperus communis*
(Juniper)
- **preparations acting on urinary tract infections**
*Vaccinium macrocarpon*
(American cranberry)
*Arctostaphylos uva-ursi*
(Bearberry)
- **preparations acting on benign prostatic hyperplasia**
*Serenoa repens* (Saw palmetto)
*Prunus africana* (African cherry)
*Urtica dioica*/*urens* (Nettle)
*Cucurbita pepo* (Pumpkin)
*Epilobium spp* (Willowherbs) or
*Solanum lycopersicum* (Tomato)
*Pinus maritima* (Maritime pine)
*Vaccinium macrocarpon* (American cranberry)
*Allium sativum* (Garlic)
*Lepidium meyenii* (Maca)
*Linum usitatissimum*
*Telfairia occidentalis* (Fluted gourd)
*Uvaria rufa* (Susung-kalabaw or Carabao teats)
- **preparations acting on erectile dysfunction**
*Panax ginseng* (Korean
ginseng)
*Lepidium meyenii* (Maca)
*Tribulus terrestris*
(Goat's head)
*Cinnamomum cassia*
(Cinnamon)
- **preparations acting on menopausal disorders**
*Cimicifuga racemosa* (Black cohosh) (Black cohosh)
*Glycine max* (Soy)
*Trifolium pratense* (Red clover)
*Vitex agnus-castus* (Vitex)
*Linum usitatissimum* (Flax)
*Hypericum perforatum* (St. John's Wort)
*Panax ginseng* (Korean ginseng)
*Ginkgo biloba* (Ginkgo)
*Crocus sativus* (Saffron)
*Valeriana officinalis* (Valerian).
- **preparations acting on the cessation of cigarette smoking**
*Cimicifuga racemosa* (Black cohosh)
*Laburnum anagyroides* (Laburnum)

4. The composition according to any one of claims 1 to 3, wherein the botanical extracts are included in the composition in an amount between 1 and 10% by weight on the total weight of the composition.

5. The composition according to any one of claims 1 to 4, for use as an adjuvant in subjects with anxiety, mental stress and mood disorders, comprising the following weight amounts:
| | |
|---|---|
| - purified water | 48-55% |
| - 1,3-propanediol | 25-35% |
| - sodium hyaluronate | 0.1-0.2% |
| - sorbitol | 0.4-0.6% |
| - sodium benzoate | 0.25-0.4% |
| - Lavender | 5-7% |
| - Hops | 5-7% |
| - Golden root | 5-7% |
| - Saffron | 2-3%. |

6. The composition according to any one of claims 1 to 4, for use as an adjuvant in subjects with sleep disorders selected from insomnia, also when linked to stress, anxiety, old age, comprising the following weight amounts:
| | |
|---|---|
| - purified water | 48-55% |
| - 1,3-propanediol | 25-35% |
| - sodium hyaluronate | 0.1-0.2% |
| - sorbitol | 0.4-0.6% |
| - sodium benzoate | 0.25-0.4% |
| - Passionflower | 4-6% |
| - Melissa | 4-6% |
| - California poppy | 4-6% |
| - Hops | 4-6%. |

7. A kit comprising one or more doses of the composition according to any one of claims 1 to 6 and a cartomizer for personal vaporizer.

## Patentansprüche

1. Medizinische Zusammensetzung zur Inhalation, die die folgenden Bestandteile umfasst:
a) Wasser 48-72 Gew.-%
b) 1,3-Propandiol 25-35 Gew.-%
c) Hyaluronsäure oder ein Salz davon 0,10-0,25 Gew.-%
d) optional ein Konservierungsmittel 0,25-0,4 Gew.-%
e) optional Sorbit 0,3-1 Gew.-%
f) eine oder mehrere pflanzliche Wirkstoffe 1-30 Gew.-%.

2. Zusammensetzung nach Anspruch 1, wobei die Hyaluronsäure oder ein Salz davon ein Molekulargewicht zwischen 1000 und 4000 kDa aufweist.

3. Zusammensetzung nach einem der Ansprüche 1 bis 2, wobei der eine oder die mehreren Wirkstoffe pflanzliche Extrakte oder Zubereitungen aus einer oder mehreren der folgenden Pflanzen sind:
- Zubereitungen mit Wirkung auf Angst und/oder Schlaflosigkeit
Valeriana officinalis (Baldrian)
Humulus lupulus (Hopfen)
Lavandula angustifolia (Lavendel)
Passiflora incarnata (Passionsblume)
Melissa officinalis (Melisse)
Matricaria chamomilla (Kamille)
Eschscholzia californica (Kalifornischer Mohn)
Centella asiatica (Centella)
Ginkgo biloba (Ginkgo)
Withania somnifera (Indischer Ginseng)
Hypericum perforatum (Johanniskraut)
- Zubereitungen mit Wirkung auf Depressionen
Hypericum perforatum (Johanniskraut)
Crocus sativus (Safran)
Salvia rosmarinus (Rosmarin)
Rhodiola rosea (Rosenwurz)
Cimicifuga racemosa (Traubensilberkerze)
Camellia sinensis (Teepflanze)
Coffea arabica (Kaffee)
Theobroma cacao (Kakao)
Paullinia cupana (Guarana)
- Zubereitungen mit Wirkung auf kognitive Störungen
Ginkgo biloba (Ginkgo)
Bacopa monnieri (Kleines Fettblatt)
Melissa officinalis (Melisse)
- Zubereitungen mit stimulierender Wirkung
Coffea arabica (Kaffee)
Camellia sinensis (Teepflanze)
Paullinia cupana (Guarana)
Ilex paraguariensis (Mate)
Cola acuminata (Cola)
Theobroma cacao (Kakao)
- Zubereitungen mit Wirkung auf Schmerzen und Entzündungen
Cannabis sativa (Hanf)
Boswellia serrata (Weihrauch)
Harpagophytum procumbens (Teufelskralle)
Tanacetum parthenium (Mutterkraut)
Commiphora myrrha (Myrrhe)
Zingiber officinale (Ingwer)
Curcuma longa (Kurkuma)
- Zubereitungen mit Wirkung auf Erkältung und Grippe
Pelargonium sidoides (Kapland-Pelargonie) oder
Spirea ulmaria (Mädesüß)
Sambucus nigra (Holunder)
Andrographis paniculata (Andrographis)
Allium sativum (Knoblauch)
Thymus vulgaris (Thymian)
Eucalyptus globulus (Eukalyptus)
Pimpinella anisum (Anis)
- Zubereitungen mit Wirkung auf Husten und Bronchitis
Althea officinalis (Eibisch)
Polygala spp. (Kreuzblume)
Grindelia robusta Nutt (Gummipflanze)
Plantago spp. (Wegerich)
Primula veris (Schlüsselblume)
Hedera helix (Efeu)
- Zubereitungen mit adaptogener Wirkung
Panax Ginseng (Koreanischer Ginseng)
Eleutherococcus senticosus (Teufelsbusch oder Sibirischer Ginseng) oder
Rhodiola rosea (Rosenwurz)
Schisandra chinensis (Chinesisches Spaltkörbchen)
Withania somnifera (Indischer Ginseng)
Uncaria tomentosa (Katzenkralle)
Astragalus membranaceus (Astragalus)
Codonopsis pilosula (Codonopsis)
Lepidium meyenii (Maca)
Ocimum tenuiflorum (Indisches Basilikum)
Pfaffia paniculata (Brasilianischer Ginseng)
Phyllantus niruri (Steinbrech)
Piper longum (Langer Pfeffer)
- Zubereitungen mit immunstimulierender Wirkung
Echinacea spp. (Echinacea)
Astragalus membranaceus (Astragalus)
Eleutherococcus senticosus (Teufelsbusch oder Sibirischer Ginseng)
Panax Ginseng (Koreanischer Ginseng)
Panax quinquefolius (Amerikanischer Ginseng)
Allium sativum (Knoblauch)
Uncaria tomentosa (Katzenkralle)
Ganoderma lucidum (Reishi-Pilz)
Grifola frondosa (Maitake-Pilz)
Coriolus versicolor (Schmetterlingstramete)
Lentinula edodes (Shiitake-Pilz)
- Zubereitungen mit Wirkung auf Bluthochdruck
Allium sativum (Knoblauch)
Olea europea (Olive)
Crataegus monogyna (Weißdorn)
Punica granatum (Granatapfel)
Hibiscus sabdariffa (Hibiskus)
Nigella sativa (Schwarzkümmel)
Linum usitatissimum (Leinsamen)
- Zubereitungen mit Wirkung auf chronische Venenschwäche
Centella asiatica (Centella)
Vaccinium myrtillus (Heidelbeere)
Vitis vinifera (Weinrebe)
Pinus maritima (Seekiefer)
Hamamelis virginiana (Hamamelis)
- Zubereitungen mit Wirkung auf Hyperlipidämie
Oryza sativa fermentiert mit Monascus purpureus (Klebreis)
Allium sativum (Knoblauch)
Cynara scolymus (Artischocke)
Commiphora mukul (Guggul)
Trigonella foenum-graecum (Bockshornklee)
Citrus bergamia (Bergamotte)
Glycine max (Soja)
Silybum marianum (Mariendistel)
- Zubereitungen mit Wirkung auf Hyperglykämie
Galega officinalis (Geißraute)
Trigonella foenum-graecum (Bockshornklee)
Gymnema sylvestre (Gymnema)
Ocimum tenuiflorum (Indisches Basilikum)
Morus alba (Weißer Maulbeerbaum)
Berberis spp. (Berberitze)
Cinnamomum verum (Zimt)
Momordica charantia (Bittermelone) oder
Allium cepa (Zwiebel)
Panax Ginseng (Koreanischer Ginseng)
- Zubereitungen mit Wirkung auf Übergewicht und Fettleibigkeit
Fucus vesiculosus (Blasentang)
Garcinia cambogia (Garcinia)
Hoodia gordonii (Hoodia)
Caralluma fimbriata (Caralluma)
Ilex paraguariensis (Mate)
Paullinia cupana (Guarana)
Amorphophallus konjac (Konjak)
Gymnema sylvestre (Gymnema)
Opuntia ficus-indica (Feigenkaktus)
Mentha piperita (Pfefferminze)
Capsicum annuum (Paprika)
Zingiber officinale (Ingwer)
Cinnamomum verum (Zimt)
- Zubereitungen mit Wirkung auf Erkrankungen der Mundhöhle
Aloe vera Gel (Aloe)
Camellia sinensis (Teepflanze)
Curcuma longa (Kurkuma)
Theobroma cacao (Kakao)
Rubus occidentalis (Schwarze Himbeere)
- Zubereitungen mit Wirkung auf Dyspepsie
Gentiana lutea (Gelber Enzian)
Zingiber officinale (Ingwer)
Capsicum annuum (Paprika)
Cannabis sativa (Hanf)
Mentha piperita (Pfefferminze)
Citrus limon (Zitrone)
Foeniculum vulgare (Fenchel)
Carum carvi (Kümmel)
Glycyrrhiza glabra (Süßholz)
- Zubereitungen mit Wirkung auf Verstopfung und Durchfall
Gummi Karaya
Guarkernmehl
Fraxinus ornus (Manna-Esche)
Tamarindus indica (Tamarinde) oder
Aloe vera (Aloe)
Rheum palmatum (Rhabarber)
Cassia angustifolia (Sennesblätter)
Rhamnus purshiana (Amerikanischer Faulbaum)
Rhamnus frangula (Faulbaum)
Agrimonia eupatoria (Odermennig) oder
Potentilla anserina (Gänsefingerkraut)
Potentilla erecta (Blutwurz)
Alchemilla vulgaris (Frauenmantel)
Quercus spp. (Eiche)
Rubus ulmifolius (Wilde Brombeere)
- Zubereitungen mit Wirkung auf entzündliche Darmerkrankungen, Reizdarm Mentha piperita (Pfefferminze)
- Zubereitungen mit Wirkung auf chronisch-entzündliche Darmerkrankungen
Curcuma longa (Kurkuma)
Boswellia serrata (Weihrauch)
Andrographis paniculata (Andrographis)
- Zubereitungen mit leberschützender Wirkung
Silybum marianum (Mariendistel)
Phyllantus niruri (Steinbrech)
Glycyrrhiza glabra (Süßholz)
Cynara scolymus (Artischocke)
- Zubereitungen mit choleretischer und cholagoger Wirkung
Curcuma longa (Kurkuma)
Cynara scolymus (Artischocke)
Peumus boldus (Boldo)
Taraxacum officinale (Löwenzahn)
- Zubereitungen mit Wirkung auf Wassereinlagerungen
Betula pendula (Hänge-Birke)
Equisetum arvense (Schachtelhalm)
Cynodon dactylon (Bermudagras)
Hieracium pilosella (Mausohr-Habichtskraut)
Juniperus communis (Wacholder)
- Zubereitungen mit Wirkung auf Harnwegsinfektionen
Vaccinium macrocarpon (Cranberry)
Arctostaphylos uva-ursi (Bärentraube)
- Zubereitungen mit Wirkung auf benigne Prostatahyperplasie
Serenoa repens (Sägepalme)
Prunus africana (Afrikanische Pflaume)
Urtica dioica/urens (Brennnessel)
Cucurbita pepo (Kürbis)
Epilobium spp. (Weidenröschen) oder
Solanum lycopersicum (Tomate)
Pinus maritima (Seekiefer)
Vaccinium macrocarpon (Cranberry)
Allium sativum (Knoblauch)
Lepidium meyenii (Maca)
Linum usitatissimum (Leinsamen)
Telfairia occidentalis (Kürbis)
Uvaria rufa (Uvaria)
- Zubereitungen mit Wirkung auf erektile Dysfunktion
Panax Ginseng (Koreanischer Ginseng)
Lepidium meyenii (Maca)
Tribulus terrestris (Erd-Burzeldorn)
Cinnamomum cassia (Zimt)
- Zubereitungen mit Wirkung auf Wechseljahresbeschwerden
Cimicifuga racemosa (Traubensilberkerze)
Glycine max (Soja)
Trifolium pratense (Rotklee)
Vitex agnus-castus (Mönchspfeffer)
Linum usitatissimum (Leinsamen)
Hypericum perforatum (Johanniskraut)
Panax Ginseng (Koreanischer Ginseng)
Ginkgo biloba (Ginkgo)
Crocus sativus (Safran)
Valeriana officinalis (Baldrian)
- Zubereitungen mit Wirkung auf die Raucherentwöhnung
Cimicifuga racemosa (Traubensilberkerze)
Laburnum anagyroides (Goldregen)

4. Zusammensetzung nach einem der Ansprüche 1 bis 3, wobei die pflanzlichen Extrakte in einer Menge zwischen 1 und 10 Gew.-%, bezogen auf das Gesamtgewicht der Zusammensetzung, enthalten sind.

5. Zusammensetzung nach einem der Ansprüche 1 bis 4 zur Verwendung als Adjuvans bei Personen mit Angstzuständen, mentalem Stress und Stimmungsschwankungen, umfassend die folgenden Mengenanteile:
- gereinigtes Wasser 48-55 %
- 1,3-Propandiol 25-35 %
- Natriumhyaluronat 0,1-0,2 %
- Sorbit 0,4-0,6 %
- Natriumbenzoat 0,25-0,4 %
- Lavendel 5-7 %
- Hopfen 5-7 %
- Rosenwurz 5-7 %
- Safran 2-3 %.

6. Zusammensetzung nach einem der Ansprüche 1 bis 4 zur Verwendung als Adjuvans bei Personen mit Schlafstörungen, ausgewählt aus Schlaflosigkeit, auch wenn diese mit Stress, Angst oder Alterung in Verbindung steht, umfassend die folgenden Mengenanteile:
- gereinigtes Wasser 48-55 %
- 1,3-Propandiol 25-35 %
- Natriumhyaluronat 0,1-0,2 %
- Sorbit 0,4-0,6 %
- Natriumbenzoat 0,25-0,4 %
- Passionsblume 4-6 %
- Melisse 4-6 %
- Kalifornischer Mohn 4-6 %
- Hopfen 4-6 %.

7. Kit, umfassend eine oder mehrere Dosen der Zusammensetzung nach einem der Ansprüche 1 bis 6 und einen Cartomizer für ein persönliches Verdampfungsgerät.

## Revendications

1. Une composition médicale destinée à être inhalée comprenant les composants suivants :
| | |
|---|---|
| a) eau | 48-72 % en poids |
| b) 1,3-propanediol | 25-35 % en poids |
| c) acide hyaluronique ou un sel de celui-ci | 0,10-0,25 % en poids |
| d) optionnellement, un conservateur | 0,25-0,4 % en poids |
| e) optionnellement, sorbitol | 0,3-1 % en poids |
| f) une ou plusieurs substances actives botaniques 1-30 % en poids. | |

2. La composition selon la revendication 1, dans laquelle l'acide hyaluronique ou un sel de celui-ci a un poids moléculaire compris entre 1000 et 4000 KDa.

3. La composition selon l'une quelconque des revendications 1 à 2, dans laquelle la ou les substances actives sont des extraits botaniques ou des préparations d'une ou plusieurs des plantes suivantes :
- **préparations agissant sur l'anxiété et/ou l'insomnie**
Valeriana officinalis (Valériane)
Humulus lupulus (Houblon)
Lavandula angustifolia (Lavande)
Passiflora incarnata (Passiflore)
Melissa officinalis (Mélisse)
Matricaria chamomilla (Camomille)
Eschscholzia californica (Pavot de Californie)
Centella asiatica (Centella)
Ginkgo biloba (Ginkgo)
Withania somnifera (Ginseng indien)
Hypericum perforatum (Millepertuis)
- **préparations agissant sur la dépression**
Hypericum perforatum (Millepertuis)
Crocus sativus (Safran)
Salvia rosmarinus (Romarin)
Rhodiola rosea (Rhodiola)
Cimicifuga racemosa (Cimicifuge)
Camellia sinensis (Théier)
Coffea arabica (Café)
Theobroma cacao (Cacao)
Paullinia cupana (Guarana)
- **préparations agissant sur les troubles cognitifs**
Ginkgo biloba (Ginkgo)
Bacopa monnieri (Bacopa)
Melissa officinalis (Mélisse)
- **préparations agissant comme stimulants**
Coffea arabica (Café)
Camellia sinensis (Théier)
Paullinia cupana (Guarana)
Ilex paraguariensis (Maté)
Cola acuminata (Cola)
Theobroma cacao (Cacao)
- **préparations agissant sur la douleur et l'inflammation**
Cannabis sativa (Chanvre)
Boswellia serrata (Boswellie)
Harpagophytum procumbens (Griffe du diable)
Tanacetum parthenium (Grande camomille)
Commiphora myrrha (Myrrhe)
Zingiber officinale (Gingembre)
Curcuma longa (Curcuma)
- **préparations agissant sur le rhume et la grippe**
Pelargonium sidoides (Géranium d'Afrique) ou Spirea ulmaria (Reine-des-prés)
Sambucus nigra (Sureau)
Andrographis paniculata (Andrographis)
Allium sativum (Ail)
Thymus vulgaris (Thym)
Eucalyptus globulus (Eucalyptus)
Pimpinella anisum (Anis vert)
- **préparations agissant sur la toux et la bronchite**
Althea officinalis (Guimauve)
Polygala spp. (Polygala)
Grindelia robusta Nutt
(Plante à gomme)
Plantago spp. (Plantain)
Primula veris (Primevère)
Hedera helix (Lierre grimpant)
- **préparations agissant comme adaptogènes**
Panax ginseng (Ginseng coréen)
Eleutherococcus senticosus (Broussaille du diable ou Ginseng de Sibérie) ou
Rhodiola rosea (Rhodiola)
Schisandra chinensis (Schisandra)
Withania somnifera (Ginseng indien)
Uncaria tomentosa (Griffe du chat)
Astragalus membranaceus (Astragale)
Codonopsis pilosula (Codonopsis)
Lepidium meyenii (Maca)
Ocimum tenuiflorum (Basilic sacré)
Pfaffia paniculata (Ginseng brésilien)
Phyllantus niruri (Phyllanthus)
Piper longum (Poivre long)
- **préparations agissant comme immunostimulants**
Echinacea spp. (Échinacée)
Astragalus membranaceus (Astragale)
Eleutherococcus senticosus (Broussaille du diable ou Ginseng de Sibérie)
Panax ginseng (Ginseng coréen)
Panax quinquefolius (Ginseng américain)
Allium sativum (Ail)
Uncaria tomentosa (Griffe du chat)
Ganoderma lucidum (Reishi)
Grifola frondosa (Maitake)
Coriolus versicolor (Tramète versicolore)
Lentinula edodes (Shiitaké)
- **préparations agissant sur l'hypertension**
Allium sativum (Ail)
Olea europaea (Olivier)
Crataegus monogyna (Aubépine)
Punica granatum (Grenadier)
Hibiscus sabdariffa (Hibiscus)
Nigella sativa (Cumin noir)
Linum usitatissimum (Lin)
- **préparations agissant sur l'insuffisance veineuse chronique**
Centella asiatica (Centella)
Vaccinium myrtillus (Myrtille)
Vitis vinifera (Vigne)
Pinus maritima (Pin maritime)
Hamamelis virginiana (Hamamélis)
- **préparations agissant sur l'hyperlipidémie**
Oryza sativa fermenté avec Monascus purpureus (Riz gluant thaï)
Allium sativum (Ail)
Cynara scolymus (Artichaut)
Commiphora mukul (Guggul)
Trigonella foenum-graecum (Fenugrec)
Citrus bergamia (Bergamote)
Glycine max (Soja)
Silybum marianum (Chardon-Marie)
- **préparations agissant sur l'hyperglycémie**
Galega officinalis (Galega)
Trigonella foenum-graecum (Fenugrec)
Gymnema sylvestre (Gymnema)
Ocimum tenuiflorum (Basilic sacré)
Morus alba (Mûrier blanc)
Berberis spp. (Épine-vinette)
Cinnamomum verum (Cannelle)
Momordica charantia (Melon amer) ou
Allium cepa (Oignon)
Panax ginseng (Ginseng coréen)
- **préparations agissant sur le surpoids et l'obésité**
Fucus vesiculosus (Varech)
Garcinia cambogia (Garcinia)
Hoodia gordonii (Hoodia)
Caralluma fimbriata (Caralluma)
Ilex paraguariensis (Maté)
Paullinia cupana (Guarana)
Amorphophallus konjac (Konjac)
Gymnema sylvestre (Gymnema)
Opuntia ficus-indica (Figuier de Barbarie)
Mentha piperita (Menthe poivrée)
Capsicum annuum (Piment)
Zingiber officinale (Gingembre)
Cinnamomum verum (Cannelle)
- **préparations agissant sur les troubles de la cavité buccale**
Aloe vera gel (Aloe)
Camellia sinensis (Théier)
Curcuma longa (Curcuma)
Theobroma cacao (Cacao)
Rubus occidentalis (Framboisier noir)
-préparations agissant sur la dyspepsie
Gentiana lutea (Gentiane jaune)
Zingiber officinale (Gingembre)
Capsicum annuum (Piment)
Cannabis sativa (Chanvre)
Mentha piperita (Menthe poivrée)
Citrus limon (Citron)
Foeniculum vulgare (Fenouil)
Carum carvi (Carvi)
Glycyrrhiza glabra (Réglisse)
- **préparations agissant sur la constipation et la diarrhée**
Gomme karaya
Gomme guar
Fraxinus ornus (Frêne à manne)
Tamarindus indica (Tamarinier) ou Aloe vera (Aloe)
Rheum palmatum (Rhubarbe)
Cassia angustifolia (Séné)
Rhamnus purshiana (Cascara)
Rhamnus frangula (Bourdaine)
Agrimonia eupatoria (Aigremoine) ou Potentilla anserina (Potentille ansérine)
Potentilla erecta (Potentille tormentille)
Alchemilla vulgaris (Alchémille)
Quercus spp. (Chêne)
Rubus ulmifolius (Ronce sauvage)
- **préparations agissant sur les pathologies inflammatoires intestinales, côlon irritable**
Mentha piperita (Menthe poivrée)
- **préparations agissant sur les maladies inflammatoires intestinales chroniques**
Curcuma longa (Curcuma)
Boswellia serrata (Boswellie)
Andrographis paniculata (Andrographis)
- **préparations agissant comme hépatoprotecteurs**
Silybum marianum (Chardon-Marie)
Phyllantus niruri (Phyllanthus)
Glycyrrhiza glabra (Réglisse)
Cynara scolymus (Artichaut)
- **préparations agissant comme cholérétiques et cholagogues**
Curcuma longa (Curcuma)
Cynara scolymus (Artichaut)
Peumus boldus (Boldo)
Taraxacum officinale (Pissenlit)
- **préparations agissant sur la rétention d'eau**
Betula pendula (Bouleau verruqueux)
Equisetum arvense (Prêle des champs)
Cynodon dactylon (Chiendent)
Hieracium pilosella (Épervière piloselle)
Juniperus communis (Genévrier)
- **préparations agissant sur les infections des voies urinaires**
Vaccinium macrocarpon (Canneberge)
Arctostaphylos uva-ursi (Busserole)
- **préparations agissant sur l'hypertrophie bénigne de la prostate**
Serenoa repens (Palmier nain)
Prunus africana (Prunier d'Afrique)
Urtica dioica/urens (Ortie)
Cucurbita pepo (Courge)
Epilobium spp. (Épilobes) ou Solanum lycopersicum (Tomate)
Pinus maritima (Pin maritime)
Vaccinium macrocarpon (Canneberge)
Allium sativum (Ail)
Lepidium meyenii (Maca)
Linum usitatissimum (Lin)
Telfairia occidentalis (Gourde nervurée)
Uvaria rufa (Uvaria rufa)
- **préparations agissant sur le dysfonctionnement érectile**
Panax ginseng (Ginseng coréen)
Lepidium meyenii (Maca)
Tribulus terrestris (Tribule terrestre)
Cinnamomum cassia
(Cannelle)
- **préparations agissant sur les troubles de la ménopause**
Cimicifuga racemosa (Cimicifuge)
Glycine max (Soja)
Trifolium pratense (Trèfle rouge)
Vitex agnus-castus (Gattilier)
Linum usitatissimum (Lin)
Hypericum perforatum (Millepertuis)
Panax ginseng (Ginseng coréen)
Ginkgo biloba (Ginkgo)
Crocus sativus (Safran)
Valeriana officinalis (Valériane)
- **préparations agissant sur l'arrêt du tabagisme**
Cimicifuga racemosa (Cimicifuge)
Laburnum anagyroides (Cytise)

4. La composition selon l'une quelconque des revendications 1 à 3, dans laquelle les extraits botaniques sont inclus dans la composition en une quantité comprise entre 1 et 10 % en poids par rapport au poids total de la composition.

5. La composition selon l'une quelconque des revendications 1 à 4, pour une utilisation en tant qu'adjuvant chez des sujets présentant de l'anxiété, du stress mental et des troubles de l'humeur, comprenant les quantités en poids suivantes :
| | |
|---|---|
| eau purifiée | 48-55 % |
| 1,3-propanediol | 25-35 % |
| hyaluronate de sodium | 0,1-0,2 % |
| sorbitol | 0,4-0,6 % |
| benzoate de sodium | 0,25-0,4 % |
| Lavande | 5-7 % |
| Houblon | 5-7 % |
| Rhodiola | 5-7 % |
| Safran | 2-3 % |

6. La composition selon l'une quelconque des revendications 1 à 4, pour une utilisation en tant qu'adjuvant chez des sujets présentant des troubles du sommeil sélectionnés parmi l'insomnie, y compris lorsque celle-ci est liée au stress, à l'anxiété ou à la vieillesse, comprenant les quantités en poids suivantes :
| | |
|---|---|
| eau purifiée | 48-55 % |
| 1,3-propanediol | 25-35 % |
| hyaluronate de sodium | 0,1-0,2 % |
| sorbitol | 0,4-0,6 % |
| benzoate de sodium | 0,25-0,4 % |
| Passiflore | 4-6 % |
| Mélisse | 4-6 % |
| Pavot de Californie | 4-6 % |
| Houblon | 4-6 % |

7. Un kit comprenant une ou plusieurs doses de la composition selon l'une quelconque des revendications 1 à 6 et un cartomiseur pour vaporisateur personnel.
